Europäisches Patentamt

(19) European Patent Office

Office européen des brevets

(11) **EP 0 864 337 A2**

## (12) EUROPEAN PATENT APPLICATION

(43) Date of publication:
16.09.1998 Bulletin 1998/38

(51) Int. Cl.$^6$: **A61N 5/10**

(21) Application number: 98104311.0

(22) Date of filing: 10.03.1998

(84) Designated Contracting States:
AT BE CH DE DK ES FI FR GB GR IE IT LI LU MC NL PT SE
Designated Extension States:
AL LT LV MK RO SI

(30) Priority: 15.03.1997 CN 97101188
08.01.1998 CN 98104506

(71) Applicant:
**Shenzhen OUR International Technology & Science Co., Ltd.**
**Shenzhen (CN)**

(72) Inventors:
• **Peng, Song Shi**
**Shenzhen (CN)**
• **Bing, Chen Zhi**
**Shenzhen (CN)**
• **Yu, Wei Kai**
**Shenzhen (CN)**
• **Hong, Tong**
**Shenzhen (CN)**
• **Zhi, Zhang Xing**
**Shenzhen (CN)**
• **Xiong, Hu Nai**
**Shenzhen (CN)**
• **Pin, Ye Zhi**
**Shenzhen (CN)**
• **Fan, Chen Rong**
**Shenzhen (CN)**
• **Ren, Ma Zhong**
**Shenzhen (CN)**

(74) Representative:
**Winter, Brandl & Partner**
**Patent- und Rechtsanwaltskanzlei**
**Alois-Steinecker-Strasse 22**
**85354 Freising (DE)**

(54) **Three-dimesional irradiation technique with charged particles of bragg peak properties and its device**

(57) The invention provides a better dosage-distributing technique and its device which utilize the Bragg peak with charged particles to carry out three-dimensional irradiation on a target area. The Bragg peak area with charged particles is selected as an irradiation unit, the unit is made to rotate round a certain rotational axle and forms a rotational irradiation voxel. By a treatment planning system, the rotational irradiation voxel is made to imitate the shape of target area. The device of the invention consists of accelerator, beam transport line, magnetic system magnifying and reducing the beam spot, beam switch installation, treatment head, treatment bed, treatment planning system and electric control system. The treatment head can swing round the central axle parallel to the ground, and it can stay at any angle. The treatment bed is equipped with a position-adjusting mechanism, it can rotate round Z axle vertical to the ground, and when the treatment head swings, the treatment bed can follow its movement, so the radiation beams drawn from the treatment head pass always a target area, and the distance from the treatment head outlet to the target area keeps constantly unvaried, in the meantime, the plane of treatment bed remains always a horizontal position.

Fig. 15

**Description**

The invention involves the field of radiotherapy, especially related to the three-dimensional irradiation techniques with charged particles of Bragg peak properties and its device.

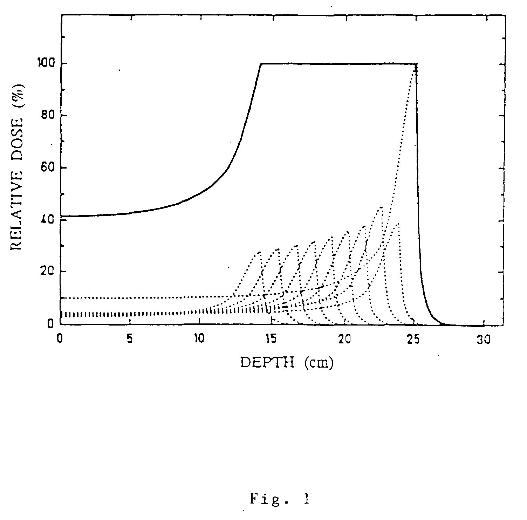

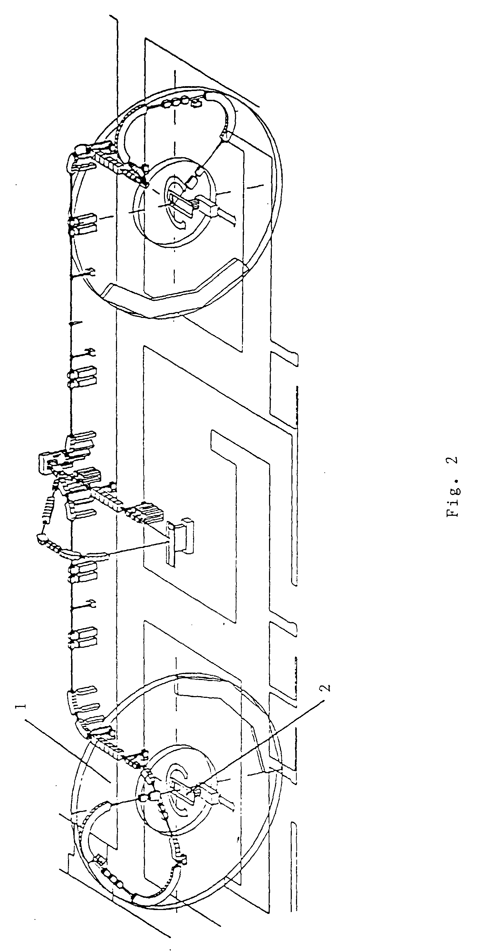

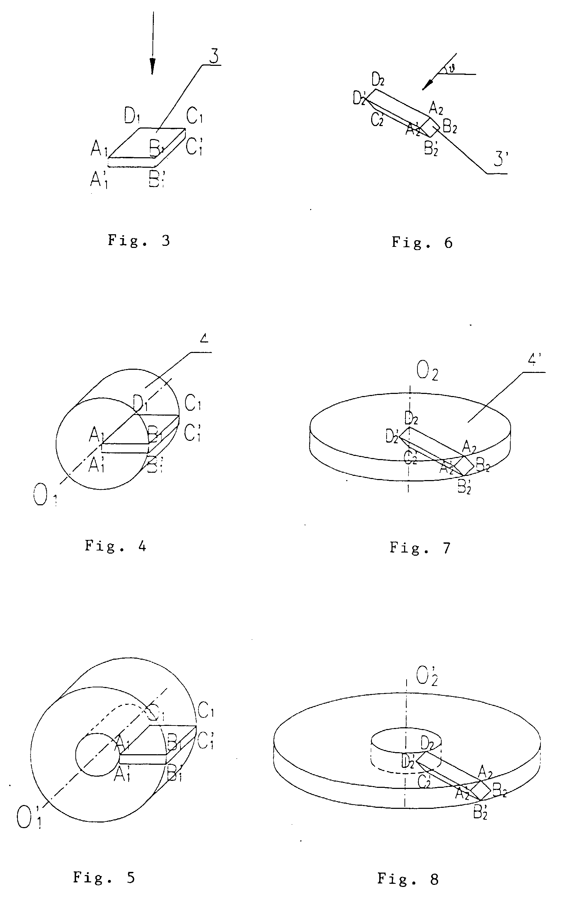

The charged particles with Bragg peak properties include protons, light ions, heavy ions and $\pi$ mesotrons etc. What is called Bragg peak refers to that, after the charged particles enter into human body, they have a well defined range, their energy is lost slowly on surface of human tissue, but concentrated their loss near the endpoint of its range, hence form a high ionized absorption peak in human body. The treatment by charged particles with Bragg peak properties is more accurate as compared with such routine radiotherapeutics as electrons, X-ray etc., so it can be used to treat the disease site in adjacent with the healthy sensitive tissues. These days the most widespread and developed one among the charged particles with Bragg peak properties in the world is proton therapy. The so-called three-dimensional conformal therapy of proton refers to that the shape of cross-section of the proton beam is made to conform that of the disease site, and meantime the Bragg peak of protons is modulated to make the shape of Bragg peak in the longitudinal region also suitable for that of the disease site. In his article "Therapy with Medium Energy Protons" in the book entitled "Advanced Techniques for Radiotherapy" (1992), Hans Blattman summarized the three-dimensional conformal techniques of protons now available, there are mainly two kinds: passive scattering and active scanning. The scattering type is that the beam extracted from an accelerator expands in its transversal vertical plane through a scattering body into evenly distributed beam flow, and collimated by collimator; longitudinally, the Bragg peak is widened by adjusting accelerator energy or increasing absorbers, so as to attain a three-dimensional treatment of the target area. The scanning type is that, in a transvansal vertical plane, the beam flow is made to scan target area by two dipole magnetic fields met at right angles; longitudinally the depth of Bragg peak is changed by adjustment of beam energy, thus irradiation is carried out one layer after another, in fact it also corresponds to the widening of Bragg peak, thus a three-dimensional conformal treatment is achieved in a target area. As shown in Fig.1, the abscissa shows the depth of protons in human body, while the ordinate shows the relative dosage of protons in human tissues; individual Bragg peak is extremely sharp, many Bragg peaks with different depths are reduplicated and reduplicated, then the widened Bragg peaks are thus obtained. Although the dosage of individual Bragg peaks is well distributed, but after the longitudinal widening of Bragg peaks the dosage received by normal tissues in the path of radiation will be multiplied for many times, and the dosage it received will also be greatly increased, Therefore, using these two kinds of three-dimensional conformal techniques of proton, we have to adopt fractional treatment, in order to avoid too large dosage for normal tissues. As shown in Fig. 2, the relatively advanced proton-therapy device now in the world use on the whole the programme of gantry. The gantry 1 rotates 360° round human body, while the treatment bed 2 rotates 360° horizontally, the combination of both can irradiate the disease site from any angle in 4 $\pi$ space. Generally speaking, 3-4 inlets are adopted for irradiation. Although making use of the techniques of gantry can carry out radiations of many inlets in a target area, yet it is still necessary to proceed irradiation for a long time in the irradiation of each inlet. Because many apparatus are needed in a gantry, it is made frequently very large, very heavy and very expensive.

The invention is aimed at providing a technique with better distributed dosage and using Bragg peaks with charged particles to carry out three-dimensional irradiation on a target area; another goal of the invention is to offer a device to realize three-dimensional irradiation technique, the structure of which is simpler than the existing scheme of gantry.

To realize the aforementioned purposes, the invention takes the following technical programme: To choose an effective Bragg peak area with charged particles as irradiation unit, let it to revolve round a certain rotary axle, thereby to form a rotational irradiation voxel. Through a treatment planning system, the rotational irradiation voxel is used to imitate the shape of a target area, so as to achieve three-dimensional irradiation of a target area.

The device of the invention includes an accelerator producing charged particles with Bragg peak properties, beam flow transport line, a magnetic system magnifying and reducing the beam spot, a beam flow switch equipment, treatment head, treatment bed, a treatment planning system and a electric control system. Among them, the treatment head consists of a magnetic system of achromatism, an adjustment system of beam energy, a collimator system for adjusting the shape of beam spot and dosage monitoring system etc., the treatment head can swing round the central axle parallel to the ground, and it can stay at any angle. The treatment bed possesses a position-adjusting mechanism, whereby the treatment bed can revolve round Z axis vertical to the ground, when treatment head swings, the treatment bed can follow its movement, the radiation beam drawn out from treatment head is made always to pass the target area, and the distance between outlet of treatment head and target area maintains constantly unvaried, at the same time the plane of the treatment bed keeps always level.

The technical advantages of the invention are that, owing to the use of Bragg peaks with charged particles, a rotational irradiation on the target area improves the dosage distribution of target area and normal tissues, In an ideal condition, we can take large dosage to kill the lesion once for all. Because it is unnecessary to have an absorber system or a scanning magetic system for conformal techniques in the device of invention, and the rotation of the gantry around the target area is changed to the swinging of treatment head and accompanied movement of treatment bed, the apparatus required for rotation are reduced, for this reason the volume of treatment head becomes smaller, the structure simpler, the weight lighter, the cost lower.

In the light of figures and specific examples in practice, the following makes a further detailed description of the invention.

Fig. 1 is a scheme of longitudinal widening on Bragg peak;

Fig. 2 is a scheme of proton therapy device now available;

Fig. 3 is a scheme of a practical example of irradiation unit in a three-dimensional irradiation technique of the invention.

Fig. 4 is a scheme of a rotational irradiation voxel formed by an irradiation unit shown in Fig. 3.

Fig. 5 is a scheme of another kind of rotational irradiation voxel formed by an irradiation unit shown in Fig. 3.

Fig. 6 is a scheme of another practical example of an irradiation unit in three-dimensional irradiation technique of the invention.

Fig. 7 is a scheme of rotational irradiation voxel formed by an irradiation unit shown in Fig. 6.

Fig. 8 is a scheme of another kind of rotational irradiation voxel formed by an irradiation unit shown in Fig. 6.

Fig. 9a, Fig. 9b - Fig. 13a, Fig. 13b are stereosocopic schemes of rotational irradiation voxel of five different forms and their corresponding irradiation units.

Fig. 14 is a scheme of irradiation path through normal tissues of three-dimensional techniques of the invention.

Fig. 15 is a scheme of therapeutic principle of three-dimensional device of the invention.

Fig. 16 is a structural scheme of a practical example in a three-dimensional device of the invention.

Fig. 17 is A-shift structural scheme of three-dimensional irradiation device in Fig. 16.

Fig. 18 is a drawing to show the mechanism principle of another practical example in three-dimensional irradiation device shown in the invention.

Fig. 19 is A'-shift structural scheme of three-dimensional irradiation device shown in Fig. 18.

It is known from Fig. 3, Fig. 4 and Fig. 5 that, in the practical example, the beam flow is proton beam, the irradiation unit 3 is a cube with a cross-section of $A_1B_1C_1D_1$, wherein $A_1B_1 = x_1$, $B_1C_1 = y_1$, the thickness is $z_1$. In general, $x_1y_1$ takes $30 \times 30mm^2$ -- $3 \times 3mm^2$.

Irradiation unit 3 can be a Bragg peak area which is not widened, in this case, there exist only single Bragg peaks, so $z_1$ takes commonly 2.5mm. The irradiation unit 3 may also be Bragg peak area widened if necessary. Because the time required for one adjustment of the depth of Bragg peak is about 40 msec, then widening for four times, namely the time needed to accomplish four adjustments, is 160 mses; when the rotational speed of beam flow is slower, for instance, 1 cycle/min, thus the degrees that the beam flow rotated in 160 msec is about 1°, in this case, it may be considered that the widened Bragg peak area revolves round the rotational axle. Taking widened Bragg peak area as irradiation unit 3, its thickness $z_1$ is then larger than 2.5mm. The rotational axle $O_1$ is parallel to the plane of treatment bed, and it coincides with $A_1 D_1$, the beam flow is made to rotate round $O_1$, thus we can obtain a cylindrical irradiation voxel 4 with a cross-section of $x_1{}^2 \pi$ and thickness of $y_1$. If $O_1$ is moved translationally to $O_1'$ along the plane of treatment bed, the distance of $O_1'$ is made from $A_1 D_1$ as $R_1$, the distance from $B_1 C_1$ is $(R_1+x_1)$, and the beam flow is made to rotate round $O_1'$, then we can obtain a circular irradiation voxel, the area of its cross-ection is $\pi(x_12+2x_1 R_1)$, and its thickness is $y_1$.

It can be seen from Fig.6, Fig. 7 and Fig. 8 that, in the present practical example, the beam flow is a proton beam which meets at angle $\theta$ with the plane of treatment bed, the irradiation unit 3' is a cube having a cross-section of $A_2B_2C_2D_2$, wherein $A_2B_2 = x_2$, $A_2D_2 = y_2$, thickness is $z_2$, and $z_2 = x_2 tg\ \theta$. $x_2y_2$ takes generally $30 \times 30mm^2$ - $3 \times 3mm^2$. The irradiation unit 3' can be a Bragg peak which is not widened, then $z_2$ takes usually 2.5mm; it may also be a widened Bragg peak area, so $z_2$ is larger than 2.5mm. The rotational axle $O_2$ is vertical to the plane of treatment bed, and passes $D_2C_2'$, the treatment bed is made to rotate round $O_2$, then we can obtain an approximate cylindrical irradiation voxel 4' with an area of cross-section of $y_2{}^2 \pi$, its thickness is $z_2 \sin \theta$. If $O_2$ is moved translationally to $O_2'$, the distance of $O_2'$ from side $D_2C_2'$ is $R_2$, the distance from another side $A_2B_2'$ is $(R_2+y_2)$, the treatment bed is made to rotate round $O_2'$, then we can obtain a circular irradiation voxel with an area of cross section of $\pi (y_2{}^2+2y_2R_2)$, and its thickness is $z_2\sin \theta$.

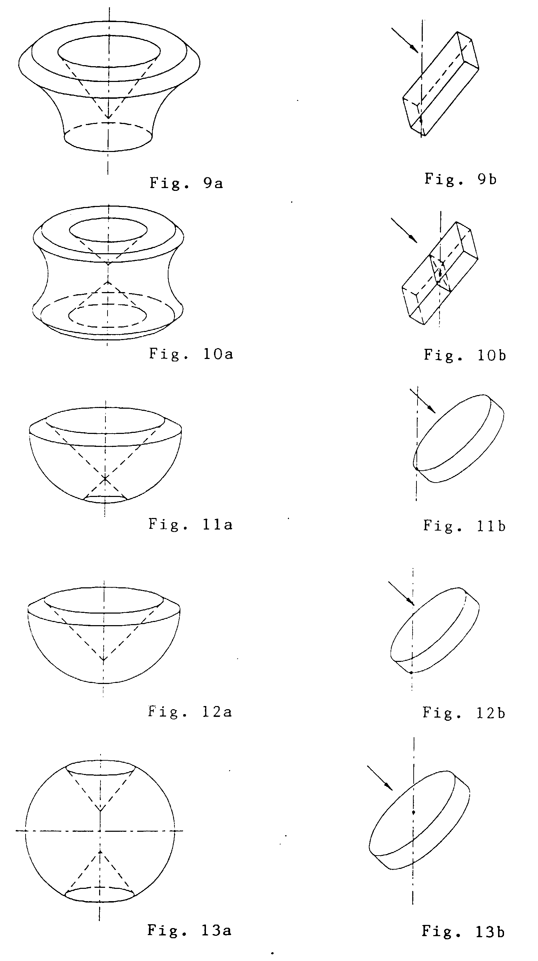

Through control of the degrees rotated by irradiation unit, adoption of different shapes of irradiation unit to rotate and change of the shape of irradiation unit in the rotational process, or selections of rotational axle at different positions relative to the irradiation unit etc., we can obtain various shapes of rotational irradiation voxel. For instance, those shown in Fig. 9a, Fig. 9b - Fig. 13a, Fig. 13b are five different shapes of rotational irradiation voxel and their corresponding irradiation units. Among them, the irradiation units in the former two cases are cubes, while the irradiation units in the latter three cases are cylinders. We can use these elemental rotational irradiation voxel to imitate various complicated shapes of target areas. We can also turn off the beam flow at certain rotational angles to protect sensitive organs.

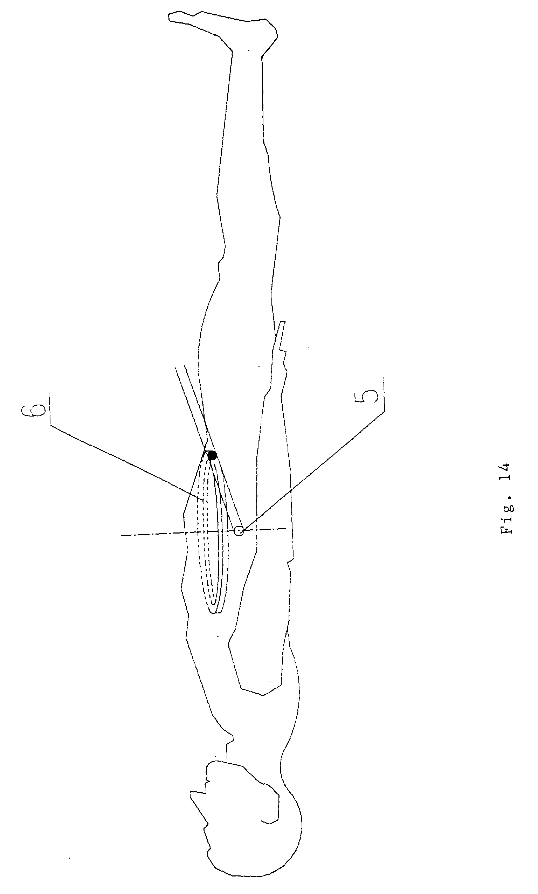

As shown in Fig. 14, the charged particle beams enter into human body, irradiation path 6 is formed in body surface, the rotational irradiation voxel needed are formed in target area 5. Using the technique of the present invention and changing the beam flow one way and long time irradiation on normal tissues into instant irradiation with constantly varied directions, thereby the beam distribution of dosage in human body are much improved.

The three-dimensional irradiation techniques of the present invention can be realized by the programme of gantry of the existing technology. But we can adopt a device with simpler structures according to the three-dimensional irradiation methods of the present invention. In the technical programme of the present invention, the rotation of gantry round the target area is changed into swinging of treatment head and accompanying movement of the treatment bed, hence providing a three-dimensional irradiation device with simpler structure, lighter weight and lower cost as compared with the programme of available gantries.

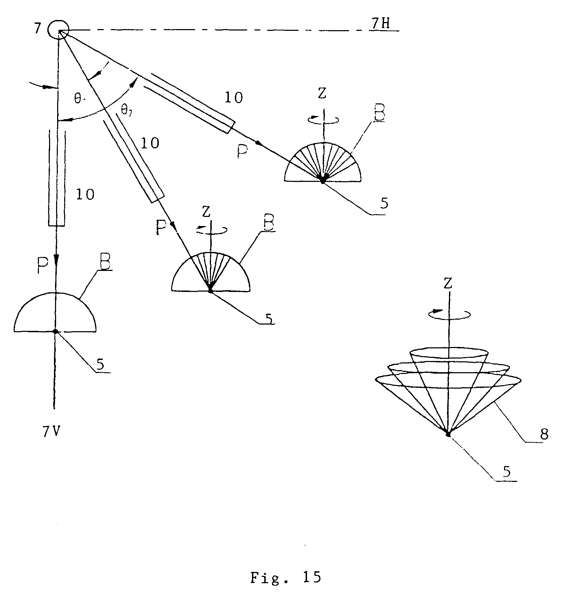

We know from Fig. 15 that, in the figure, 7 is swing axle, 7H is horizontal line, 7V is plumb line, P is proton beam, Z axle is rotational axle of treatment bed vertical to the ground, B is the body surface of a patient, 5 is target area, 8 is the cone through which the proton beam in the process of treatment passes within the patient's body. During treatment, the target areas are decomposed into irradiation voxels one by one firstly in accordance of the shape of target area, and a treatment plan is formulated. Then, based on the treatment plan and under the control of electric system, stay a moment when the treatment head 10 swings from 0° to $\theta_1$ (the incident angle of proton beam is $\theta_1$), and let the treatment bed (not shown in the figure) rotate at a slower speed of rotation round Z axle for one cycle (i.e.360°), for example at a speed slower than 1 cycle/min., thus a rotational irradiation voxel is formed. In the process of rotation, the plane of treatment bed maintains level. When the treatment head 10 swings again from $\theta_1$ to $\theta_2$, stay for a while to let treatment bed rotate one cycle again, thereby another rotational irradiation voxel is formed. At certain rotational angle, the beam is turned off by a beam switch installation, in order to protect sensitive organs, and so on and so forth, a three-dimensional irradiation on the entire target area can be realized in such a manner.

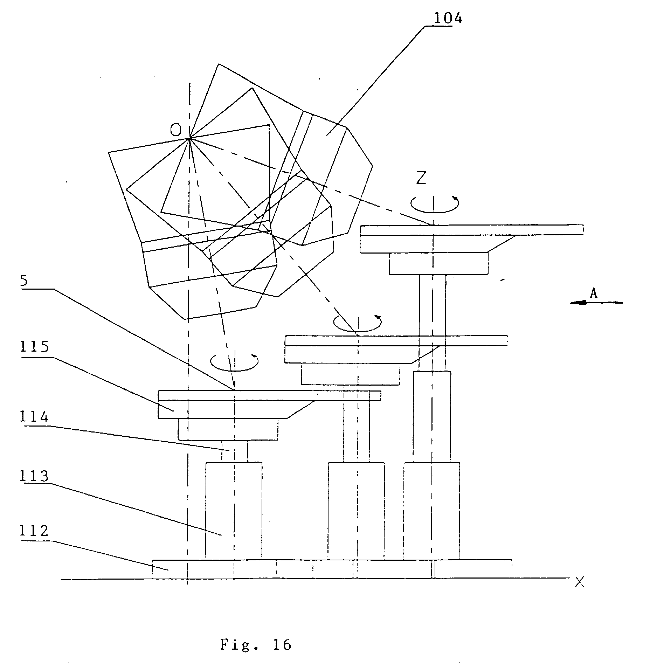

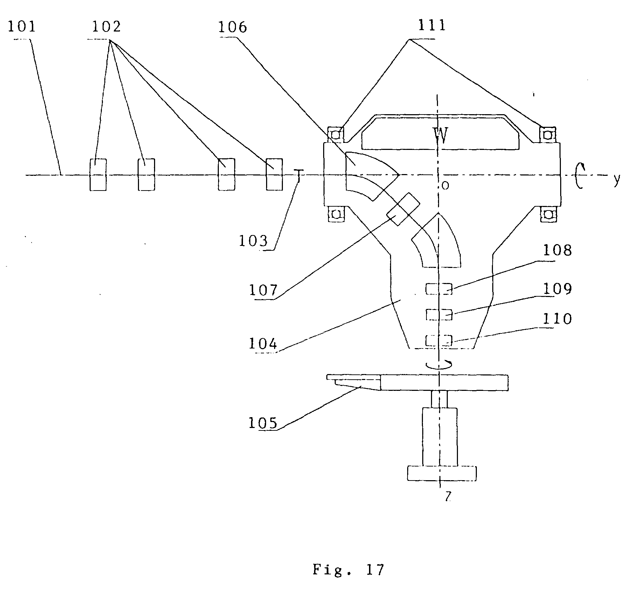

We know from Fig. 16 and Fig. 17 that, in the present practical example, the proton beams are extracted from proton synchronic accelerator, and the energy is adjustable between 250MeV - 70 MeV. After extracted, the size of proton beam is adjusted by the beam transport line 101 as well as the magnetic system 102 magnifying and reducing beam spot, and a beam switch installation 103 is equipped to guarantee the safety during treatment, then it enters into treatment head 104, the central axle of beam entering into treatment head 104 is parallel to the ground. The treatment head 104 includes two 45° deflection magnets 106 as well as one achromatism consisting of quadrupole 107, the beam flow energy-adjusting system 108, the collimator system 110 adjusting the shape of beam spot and dosage monitor system 109 etc. The treatment head 104 uses a double supporting mechanism 111 for sustain, thus it may greatly reduce deflection. The central axle of beam flow drawn out from treatment head 104 is made vertical to the central axle of the beam flow entering into the treatment head 104, and intersected at O point. The treatment head 104 can do simple pendulum movement not surpassing ± 45° round O point, and it can stay at any angle. Owing to a great reduction of apparatus inside treatment head 104 as compared with the gantry in the existing technology, the control on the swinging of treatment head 104 is simpler, the bob-weight needed is decreased in comparison with that of gantry, thus the volume is cut down, and the cost is lowered.

A scatter system required for scattering type of three-dimensional conformal irradiation or a scanning magnetic system required for scanning three-dimensional conformal irradiation can also be installed in the rapy-head 104, in order to give consideration of conformal irradiation of the disease site.

The treatment bed 105 includes pedestal 112, bed-axle and bed-body 115. Among them, the pedestal comprises an X-shift guiding mechanism, it can move along X direction; the bed-axle contains Z-shift elevator 114 and rotator 113, it can lift up and down along Z direction and rotate round Z axle; the bed-body 115 comprises a guiding mechanism, it can move along X and Y directions, the plane of bed-body is parallel to the ground. The treatment bed 105 can also increase one or two degrees of freedom, i.e., it rotates round the X-shift axle or Y-shift axle, in order to help adjustment during irradiation.

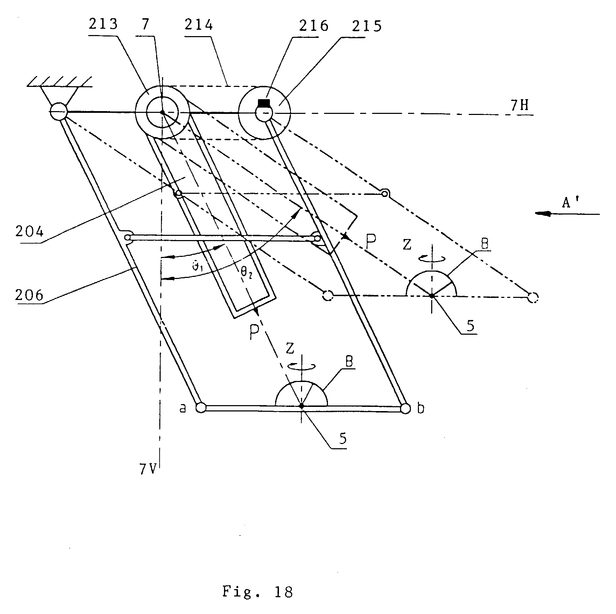

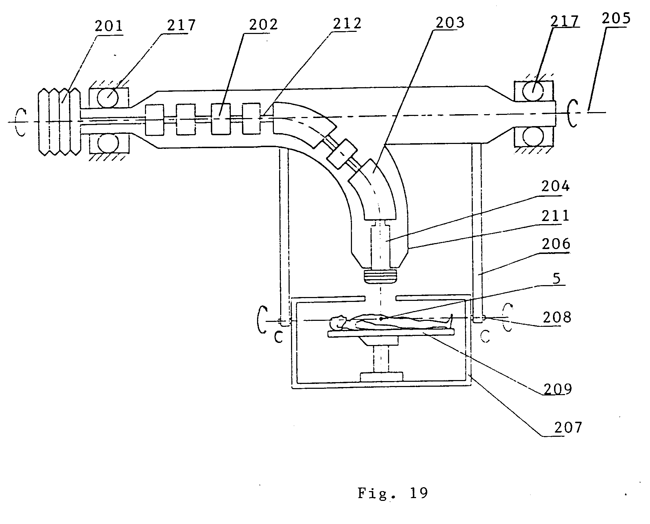

We know from Fig. 18 and Fig 19 that, in another practical example of three-dimensional irradiation device of the invention, a parallelogram connecting rod mechanism 206 is used, the treatment head 204 and the parallelogram connecting rod mechanism 206 are rigidly fixed together. The treatment head 204 is driven by a stepping motor in order to realize accurate localization and graduation. The stepping motor drives turbine pairs 213, and makes the chief axle 205 rotate for driving treatment head 204, meantime the parallelogram connecting rod mechanism is driven by chain 214, sprocket 215, square key 216, in this manner, while the treatment head 204 swings, the horizontal connecting rod ab moves steadily and at even speed together. We know from the properties of parallelogram connecting rod mechanism that no matter what angle it swings, the proton beams drawn from treatment head 204 intersects always connecting rod ab at a constant point, namely the distance between target area 5 and beam drawing outlet will never change.

The proton beams are drawn out from a cyclotron and the energy is adjustable between 70-235 MeV. After drawing out, it enters into a treatment head 204 via beam transport line, four quadrupoles 202 magnifying and reducing the beam spot, as well as two 45° deflection magnets 203, the aforementioned parts, such as quadrupole 202, deflection magnet 203, beam tube 212, treatment head 204 as well as shielding 211 etc. are all rigidly fixed with swing span 206, they can swing at ± 65° in the vertical plane relative to the plumb line by take horizontal beam axle line 205 as rotational axis, and they can stay at any angle. The swing axle uses a double structural support 217 for sustain, thus it may greatly reduce deflection and increase therapeutic accuracy. At the upperstream of quadrupole 202, there is a rotational joint 201 of hermetic seal, so it guarantees the rotational movement of the entire swing mechanism not to be restricted. The treatment bed 209 with four degrees of freedom is fixed on the base plate of cradle 207, the cradle 207 and swing span 206 are joined in the manner of hinge 208, forming a parallelogram mechanism, its hinge points c, d are all located on the same level, it is called below for short as hinge level. Thus, whatever angle it swings, the base plate of the cradle and treatment bed 209 keep always a horizontal position, namely in the process of treatment the patient maintains constantly a lying posture, without any inclination. On the hinge level, there installed x, y laser locators and X-ray locator, these locators and the same locators on the treatment head 204 make up a three-dimensional localizing monitor system. Before treatment, the target area 5 is positioned by these locators and the three-dimensional movement of treatment bed 209, at the intersecting point 210 of proton beam and hinge level. In this manner, no matter what angle the treatment head 204 swings in the process of treatment, the proton beam and hinge level are always intersected in the target area 5, and the distance between the beam drawing outlet 211 and the target area 5 is kept unvaried, the whole course of treatment needs consequently no repositioning.

The present invention guarantees a synchronous movement of treatment head 204 and treatment bed 209, and assures a horizontal state of treatment bed in the process of swinging by way of the rigid connection of treatment head 204 and swing span 206, as well as the hinged joint of cradle 207 and swing span 206 that form a parallelogram mechanism.

The swinging weight of the cradling proton therapy device is less than 25 tons, while the installation including swing route is totally about 6 meters in height. Its volume and weight are much smaller than those adopted generally now by gantry, the most important thing is to use a pattern of swing cradling treatment, which can precisely localize and treat tumors. It not only greatly increases the therapeutic power of the device, lowers the cost effectively, in the meantime the patient is under a short period of treatment, and the utility of the installation is high, but it also enhances the quality of proton treatment to a brand new level, and enormously promotes the popularization of proton therapy on a global scale.

## Claims

1. A three-dimensional irradiation technique with charged particles of Bragg peak properties, it is characterized by: selection of not widened Bragg peak area of charged particles as irradiation unit (3 or 3'), the irradiation unit is made to rotate round a certain rotational axle ($O_1$ or $O_2$), thereby a rotational irradiation voxel (4 or 4') is formed; through a treatment planning system, the rotational irradiation voxel (4 or 4') is used to imitate the shape of target area.

2. A three-dimensional irradiation technique with charged particles of Bragg peak properties, it is characterized by: widening of Bragg peak area of charged particles, selection of widened Bragg peak area as irradiation unit (3 or 3'); in the meantime the irradiation unit (3 or 3') is made to rotate round a certain rotational axle ($O_1$ or $O_2$), the speed of rotation is slower than that of adjusting the depth of the Bragg peak, thus a rotational irradiation voxel (4 or 4') is formed; through a treatment planning system, the rotational irradiation voxel (4 or 4') is used to imitate the shape of target area.

3. According to the three-dimensional irradiation technique mentioned in Claim 1 or 2, it is characterized by: in the process of rotation of irradiation unit (3 or 3') round the rotational axle ($O_1$ or $O_2$), the shape of irradiation unit (3 or 3') is changed.

4. According to the three-dimensional irradiation technique mentioned in Claim 1 or 2, it is characterized by: rotational axle $O_1$ of the mentioned irradiation unit (3) is parallel to the plane of treatment bed.

5. According to the three-dimensional irradiation technique of protons mentioned in Claim 1 or 2, it is characterized by: rotational axle $O_2$ of the mentioned irradiation unit (3') is vertical to the plane of treatment bed.

6. According to the three-dimensional irradiation technique mentioned in Claim 3, it is characterized by: rotational axle $O_1$ of the mentioned irradiation unit (3) is parallel to the plane of treatment bed.

7. According to the three-dimensional irradiation technique mentioned in Claim 3, it is characterized by: rotational axle $O_2$ of the mentioned irradiation unit (3') is vertical to the plane of treatment bed.

8. A three-dimensional irradiation device for realizing the technique mentioned in Claim 1 or 2, including the device that can produce charged particles with Bragg peak properties, the beam transport line (101), treatment head (104) and treatment bed (105); the treatment bed (105) possesses a mechanism for adjusting positions, it can make three-dimensional movement and rotate round Z axle; it is characterized by: the treatment head (104) can swing round the central axle parallel to the ground, and it can stay at any angle.

9. According to the three-dimensional irradiation device mentioned in Claim 8, it is characterized by: the mentioned treatment head (104) adopts a double supportiong mechanism (111) to sustain.

10. A three-dimensional irradiation device for realizing the technique mentioned in Claim 1 or 2, including the device that can produce charged particles of Bragg peak properties, hermetically sealed rotational joint (201), quadrupole (202) magnifying and reducing the beam spot, beam tube (212), deflection magnet (213), treatment head (214), swing span (206), cradle (207) as well as treatment bed (209) which is fixed on the cradling base plate; the mentioned quadrupole (202), deflection magnet (203), beam tube (212) and treatment head (204) are all fixed rigidly together with swing span (206), and they can swing by taking the horizontal beam axle (205) as rotational axle, and can stay at any angle; the cradle (207) and swing span (206) are joined in the manner of hinge, all its hinge points (c, d) are on the same horizontal plane, the swing span (206) and cradle (207) form a parallelogram mechanism on the rotational plane in such a way that whatever angle the swing span (206) swings it always keeps all planes with the hinge points and the plane of treatment bed maintain a horizontal position; the beams drawn out from treatment head (204) pass constantly over the intersecting point (210) where the rotational Z axle of treatment bed (209) vertical to the ground meets with the horizontal plane of hinge points, and the distance between the beam outlet and the intersecting point (210) is unvaried.

11. According to the three-dimensional irradiation device mentioned in Claim 10, it is characterized by: the mentioned swing axle (205) adopts a double supporting mechanism (217) to sustain.

12. According to the three-dimensional irradiation device mentioned in Claim 10, it is characterized by: on the horizontal plane where exist the mentioned hinge points are installed x, y laser locators and X-ray locator, these locators and the same locators on the treatment head (204) form a three-dimensional localization monitor system.

13. According to the three-dimensional irradiation device mentioned in Claim 10, it is characterized by: the mentioned treatment head (204) is driven by a stepping motor, and at the same time the swing span (206) is driven to rotate synchronously by turboshaft turbine (213), chain (214), sprocket (215) and square key (216).

Fig. 1

Fig. 2

Fig. 3

Fig. 6

Fig. 4

Fig. 7

Fig. 5

Fig. 8

Fig. 9a

Fig. 9b

Fig. 10a

Fig. 10b

Fig. 11a

Fig. 11b

Fig. 12a

Fig. 12b

Fig. 13a

Fig. 13b

Fig. 14

Fig. 15

Fig. 16

Fig. 17

EP 0 864 337 A2

Fig. 18

Fig. 19